# EUROPEAN PATENT APPLICATION

(11) **EP 4 851 626 A1**
(43) Date of publication of application: **09.12.2026**
(21) Application number: 25180519.8
(22) Date of filing: 03.06.2025
(51) Int. Cl.: G01N 33/53, G01N 33/543, G01N 33/566, G01N 33/66, C07K 14/705

(54) **A METHOD OF DETECTING OR PURIFYING A GLYCOSYLATED ANALYTE AND RELATED SYSTEMS**

(71) Applicant: Fraunhofer-Gesellschaft zur Förderung der angewandten Forschung e.V., 80686 München (DE)
(72) Inventor: Safarian, Schara, 60596 Frankfurt am Main (DE); Kannt, Aimo, 60596 Frankfurt am Main (DE); Ernst, Andreas, 60596 Frankfurt am Main (DE); Rasoulinejad, Samaneh, 60596 Frankfurt am Main (DE); Seifert, Michelle, 60596 Frankfurt am Main (DE); Kollenkirchen, Tim, 60596 Frankfurt am Main (DE)
(74) Representative: Kuttenkeuler, David

(57) **Abstract**

The invention provides devices and systems for the detection, quantification and purification of glycosylated proteins, both endogenously glycosylated proteins as well as *in-vitro* glycosylated proteins. The invention is based on a capture probe specifically binding glycosylation molecules (glycans) which was designed on the basis of the extracellular domain of Asialoglycoprotein receptor (ASGPR) 1 or 2.

## Description

### FIELD OF THE INVENTION

The invention provides devices and systems for the detection, quantification and purification of glycosylated proteins, both endogenously glycosylated proteins as well as *in-vitro* glycosylated proteins. The invention is based on a capture probe specifically binding glycosylation molecules (glycans) which was designed on the basis of the extracellular domain of Asialoglycoprotein receptor (ASGPR) 1 or 2.

### DESCRIPTION

Glycosylation represents a fundamental post-translational modification of proteins, wherein carbohydrate moieties (glycans) are covalently attached to specific amino acid residues. This modification occurs predominantly within eukaryotic cells, in particular within the endoplasmic reticulum and Golgi apparatus, and plays a critical role in influencing protein folding, stability, solubility, cellular localization, and biological activity. Glycosylation is further recognized as a key determinant in mediating cell-cell interactions, immune responses, and protein clearance rates.

Two principal forms of glycosylation are commonly observed: N-linked and O-linked glycosylation. N-linked glycosylation involves the attachment of glycans to the nitrogen atom of asparagine residues, typically within the consensus sequence Asn-X-Ser/Thr (where X is any amino acid except proline), and is initiated in the endoplasmic reticulum with subsequent processing in the Golgi apparatus. O-linked glycosylation, by contrast, entails the attachment of glycans to the hydroxyl group of serine or threonine residues, and is generally initiated in the Golgi.

In the context of recombinant protein expression and therapeutic protein development, glycosylation is of particular relevance, as the presence, absence, or structure of glycan moieties may directly affect the pharmacological properties of a given protein, including its biological activity, in vivo half-life, and immunogenic potential. For example, the glycosylation profile of monoclonal antibodies may influence effector functions such as antibody-dependent cellular cytotoxicity (ADCC). Accordingly, the ability to control, analyze, and characterize glycosylation patterns is of critical importance in the production and regulatory approval of biopharmaceuticals. Established analytical techniques for assessing glycosylation include, but are not limited to, mass spectrometry, chromatography, and lectin-based binding assays.

Artificial glycosylation of proteins may be accomplished through a variety of established methodologies, including enzymatic, chemical, and cell-based approaches. These techniques enable the introduction, modification, or control of glycan structures on proteins for various applications, including therapeutic development, structural analysis, and functional studies.

Enzymatic glycosylation involves the use of glycosyltransferases or endoglycosidases to attach defined glycan structures to specific sites on a protein in vitro. This method allows for high specificity and reproducibility and is often employed to produce homogenous glycoforms or to remodel glycosylation patterns following protein expression. Chemical glycosylation, by contrast, utilizes site-specific or random covalent conjugation of glycans or glycan mimetics to amino acid side chains through chemically reactive groups. Techniques such as click chemistry are commonly employed in this context to enable efficient and selective glycan attachment, including the incorporation of non-natural sugar analogs.

Chemoenzymatic methods combine the precision of chemical site-modification with the selectivity of enzymatic glycan transfer, thereby offering a flexible and efficient strategy for constructing structurally defined glycoproteins. In addition, cell-based glycoengineering approaches are widely used for large-scale production of glycoproteins with specific glycosylation profiles. These methods typically involve the use of genetically modified host cells-such as Chinese hamster ovary (CHO) cells, human embryonic kidney (HEK293) cells, yeast, or engineered bacterial systems-wherein the endogenous glycosylation pathways are altered or supplemented with exogenous enzymes to produce desired glycoforms.

Despite the utility of artificial glycosylation methods, each approach presents inherent limitations. Enzymatic glycosylation is constrained by the limited availability and substrate specificity of glycosyltransferases, as well as the high cost of enzymatic reagents. Chemical glycosylation often suffers from poor site specificity and may require reaction conditions that can compromise protein integrity, while also limiting the complexity of glycan structures that can be introduced. Chemoenzymatic strategies, though more precise, involve multi-step processes that can be labor-intensive and challenging to optimize. Cell-based glycoengineering approaches, while scalable, may produce heterogeneous or non-human glycan structures, necessitating complex genetic modifications and raising regulatory concerns in therapeutic applications. These limitations underscore the need for improved glycosylation methods that are efficient, site-specific, scalable, and capable of producing biologically relevant glycoforms.

Recently, the present inventors developed a new platform for a sortase reaction based attachment of carbohydrates to proteinaceous target molecules. The object of the present invention was in this context to provide assay systems to test glycosylation efficiency and to purify products obtained from glycosylation.

### BRIEF DESCRIPTION OF THE INVENTION

The above object was solved generally, and by way of brief description, the main aspects of the present invention, which can be described as follows:

In **a first aspect,** the invention pertains to an assay device for the detection and/or quantification of glycan modified analytes, comprising a capture protein composed of a soluble extracellular domain of Asialoglycoprotein receptor (ASGPR) 1 or 2, or a protein having at least 80% sequence identity to an extracellular domain of ASGPR1 or ASGPR2; and wherein the capture protein comprises at least a Carbohydrate recognition domain (CRD) of ASGPR1 or ASGPR2.

In **a second aspect,** the invention pertains to a method for detecting and/or quantifying the glycosylation of a target analyte, the method comprising:
(a) Providing the assay device of the invention,
(b) Contacting the capture protein with an analyte sample;
(c) Optionally, removing unbound material (washing step);
(d) Detecting the presence and the amount of analyte bound to the capture protein.

In **a third aspect,** the invention pertains to an assay kit, comprising the assay device of the invention, and optionally:
(a) One or more buffers and/or reagents
(b) A first detection antibody and optionally a second detection antibody.

In **a fourth aspect,** the invention pertains a use of an capture protein recited in the previous aspects for quantifying the in-vitro glycosylation of a target molecule and/or for purifying a glycosylated target molecule.

In **a fifth aspect,** the invention pertains to a purification device for glycan modified analytes, comprising a capture protein which is composed of a soluble extracellular domain of Asialoglycoprotein receptor (ASGPR) 1 or 2, or a protein having at least 80% sequence identity to an extracellular domain of ASGPR1 or ASGPR2; and wherein
the capture protein comprises at least a Carbohydrate recognition domain (CRD) of ASGPR1 or ASGPR2;
wherein the capture protein is as defined in any one of the preceding claims.

**In a sixth aspect,** the invention pertains to a method purifying a glycosylated molecule, the method comprising:
(a) Providing the purification device of the invention;
(b) Contacting the capture protein with an unpurified sample containing the glycosylated molecules, preferably under conditions allowing for a specific binding of the glycosylated protein to the capture protein;
(c) Optionally, removing unbound material (washing step), preferably under conditions retaining bound glycosylated protein to the capture protein;
(d) Eluting the glycosylated protein from the capture protein under conditions that allow for a release of the binding interaction between the capture protein and the glycosylated molecule,
(e) Obtaining from the elute in (d) a purified composition of the glycosylated protein.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The terms "contacting", "contacted" and the like as used herein in relation to two or more substances broadly include conditions which allow contact or interaction between the two or more substances whether in solution or in solid phase. For example, contacting two or more substances may comprise mixing together the two or more substances in solution or adding the two or more substances to the same substrate. The terms do not necessarily require actual physical contact between the two or more substances. The terms "incubating", "incubated" and the like as used herein in relation to two or more substances means contacting two or more substances for a sufficient duration of time to allow interaction between the two or more substances to take place. No specific temperature requirements are implied by the terms "incubating", "incubated" and the like unless otherwise indicated.

The terms "bind," "binding,", "bound" and the like as used herein broadly include to any type of immobilization, formation of a complex, fixation, or attachment between two or more substances, between a substance and one or more surfaces, or a combination thereof, regardless of the mechanism or mechanisms of attachment involved. Examples of mechanisms include, but are not limited to, formation of complexes (e.g. protein-protein complex, antibody-antigen complex, non-antibody protein-antigen complex etc.), covalent bonding, ionic bonding, electrostatic forces, hydrogen bonding, van der Walls attraction, hydrophobic effect, absorption and adsorption.

The term "affinity" as used herein refers to an attraction or non-random interaction between two molecules or parts thereof, for example, between cellulose and cellulose-binding domain. The term "micro" when used as a unit prefix (e.g. 1 micro (p)) generally denotes a factor of 10"6. When used in relation to dimensions, the term is to be interpreted broadly to include dimensions from about 1 micron to about 1000 microns.

The term "particle" as used herein broadly refers to a discrete entity or a discrete body. The particle described herein can include an organic, an inorganic or a biological particle. The particle used described herein may also be a macro- particle that is formed by an aggregate of a plurality of sub-particles or a fragment of a small object. The particle of the present disclosure may be spherical, substantially spherical, or non-spherical, such as irregularly shaped particles or ellipsoidally shaped particles. In some examples, the term "size" when used to refer to the particle broadly refers to the largest dimension of the particle. For example, when the particle is substantially spherical, the term "size" can refer to the diameter of the particle; or when the particle is substantially non-spherical, the term "size" can refer to the largest length of the particle. In some examples, the term "size" when used to refer to the particle broadly refers to the weight of the particle. For example, the "size" of a protein may be described using its molecular mass in kilodaltons (kDa) unit.

The terms "coupled" or "connected" as used in this description are intended to cover both directly connected or connected through one or more intermediate means, unless otherwise stated.

The term "associated with", used herein when referring to two elements refers to a broad relationship between the two elements. The relationship includes, but is not limited to a physical, a chemical or a biological relationship. For example, when element A is associated with element B, elements A and B may be directly or indirectly attached to each other or element A may contain element B or vice versa.

The term "adjacent" used herein when referring to two elements refers to one element being in close proximity to another element and may be but is not limited to the elements contacting each other or may further include the elements being separated by one or more further elements disposed therebetween.

The terms "substrate," "support," or "matrix" refer to a solid or semi-solid material that provides a physical surface or structural framework onto which biological, chemical, or physical interactions can occur, including but not limited to the immobilization of molecules, cells, or reagents, or the facilitation of binding, separation, or detection processes.

For clarity, the various preferred embodiments and definitions herein provided in context of one aspect shall be understood, where it makes technical sense, to equally apply to other aspects of the invention as provided. The disclosure of an aspect is particular not to be understood to be read in isolation of the other aspects. Rather, the disclosure of each aspect is to be read and understood in context of the full disclosure of all aspects as well as the figures, examples and introcution.

In **a first aspect,** the invention pertains to an assay device for the detection and/or quantification of glycan modified analytes, comprising a capture protein which is composed of a soluble extracellular domain of Asialoglycoprotein receptor (ASGPR) 1 or 2, or a protein having at least 80%, 85%, 90%, 95%, 97%, 98% or, most preferably, 99% sequence identity to an extracellular domain of ASGPR1 or ASGPR2; and wherein the capture protein comprises at least a carbohydrate recognition domain (CRD) of ASGPR1 or ASGPR2.

In a **second aspect,** the invention pertains to a method for detecting and/or quantifying the glycosylation of a target analyte, the method comprising:
(a) Providing the assay device of the invention,
(b) Contacting the capture protein with an analyte sample;
(c) Optionally, removing unbound material (washing step);
(d) Detecting the presence and the amount of analyte bound to the capture protein.

In an additional aspect, the invention also pertains to the herein below described isolated capture protein, or a nucleic acid or nucleic acid construct such as a vector, encoding the capture protein.

In various embodiments, there is provided a method of detecting a glycosylated analyte (the analyte), the method comprising capturing the analyte onto a substrate with a capture protein comprising a carbohydrate recognition domain (CRD) of ASGPR1 or ASGPR2. In various embodiments, there is provided a method of detecting an analyte in a sample, the method comprising incubating the sample with a reporter agent and/or a capture protein before applying the sample to a substrate. In various embodiments, there is provided a method of detecting the analyte in a sample, the method comprising: applying the sample to a substrate to allow said analyte, if present in said sample, to be captured onto said substrate by a capture protein comprising a carbohydrate recognition domain (CRD) of ASGPR1 or ASGPR2, wherein said capture protein is: (i) incubated with said analyte sample prior to the applying step; and/or (ii) immobilised on said substrate prior to the applying step; and determining a presence or absence of said analyte captured on said substrate by said capture protein. Methods for determining whether an analyte is captured on a substrate that are known in the art may be suitably applied in the disclosed method. For example, a detectable reporter agent may be used to bind to the analyte or the capture protein on the substrate and the signal effected by the reporter agent may indicate the presence or absence of analyte on the substrate. Detecting an analyte may comprise detecting a presence, an absence, an amount/concentration, or a relative amount/concentration of an analyte. The detection may be qualitative, quantitative or semi quantitative. For example, an amount/concentration or a relative amount/concentration of an analyte can be determined by measuring an intensity of the signal or a rate of production of the signal effected by the reporter agent, and then comparing the intensity or rate with that effected by a control sample or a comparative sample containing a known amount of analytes. If the intensity or rate of the signal obtained from the sample is greater than that obtained from the control sample or the comparative sample in a non-competitive binding assay, this may be indicative that the sample contains a greater amount/concentration of the analyte than the control sample or the comparative sample. The amount/concentration of the analyte in the sample may be determined by calculating the ratio of the intensity or rate obtained from the sample to the intensity or rate obtained from the control sample or the comparative sample.

The method of detecting an analyte may also comprise detecting two or more analytes, or a plurality of analytes. For example, two or more different analytes, or a plurality of different analytes, may be detected by use of different reporter agents, or by capturing the different analytes onto different spatial regions on the substrate or to different substrate entities.

The term "analyte", as used herein broadly refers to a substance which is to be detected/measured and which is a carbohydrate or a molecule that is glycosylated, hence carries or is attached to a carbohydrate. Examples of analytes include, but are not limited to, prokaryotic or eukaryotic cells of any type, microorganisms, bacteria, pathogens, viruses, prions, organic compounds, lipids, carbohydrates, hormones, antibodies, antigen-binding proteins, peptides, amino acids, nucleic acids/polynucleotides such as deoxyribonucleic acid (DNA) and ribonucleic acids (RNA), steroids, vitamins, drugs (including those administered for therapeutic purposes as well as those administered for illicit purposes), drug intermediates, toxins, chemicals, pesticides, pollutants, metal, heavy metal, metal ions and metabolites, portions, fragments and extracts of the aforementioned materials and combinations thereof. In some embodiments, the analyte comprises a virus. In some embodiments, the analyte comprises a viral protein. In some embodiments, the analyte, or portion thereof, is antigenic or haptenic having at least one determinant site, or is a member of a binding pair.

The carbohydrate or saccharide according to the present invention is not limited by its source or origin, and encompasses those obtained from natural origins including those from human and other mammalian sources, those produced by genetically engineered animal cells, plant cells, microorganisms, and other cells, those enzymatically manufactured, those manufactured by fermentation processes, those artificially synthesized by chemical processes and others. The carbohydrate or saccharide may encompass monosaccharides, disaccharides, oligosaccharides, and polysaccharides. Examples of N-acyl-2-amino monosaccharides include, but are not limited to, N-acetylglucosamine, N-acylglucosamine, N-acetylgalactosamine, N-acylgalactosamine, N-acetylmannosamine, N-acylmannosamine, N-acetyllallosamine, N-acylallosamine, (2S,3S,4R,5R,6R)-5-acetamido-3,4,6-trihydroxytetrahydro-2H-pyran-2-carboxylic acid, (2S,3R,4R,5R,6R)-5-acetamido-3,4,6-trihydroxytetrahydro-2H-pyran-2-carboxylic acid, (2S,3S,4S,5R,6R)-5-acetamido-3,4,6-trihydroxytetrahydro-2H-pyran-2-carboxylic acid, (2R,3S,4R,5R,6R)-5-acetamido-3,4,6-trihydroxytetrahydro-2H-pyran-2-carboxylic acid, (2S,3S,4R,5R,6R)-5-acyl-3,4,6-trihydroxytetrahydro-2H-pyran-2-carboxylic acid, (2S,3R,4R,5R,6R)-5-acyl-3,4,6-trihydroxytetrahydro-2H-pyran-2-carboxylic acid, (2S,3S,4S,5R,6R)-5-acyl-3,4,6-trihydroxytetrahydro-2H-pyran-2-carboxylic acid, (2R,3S,4R,5R,6R)-5-acyl-3,4,6-trihydroxytetrahydro-2H-pyran-2-carboxylic acid, (2S,3S,4R,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, (2S,3R,4R,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, (2S,3S,4S,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, (2R,3S,4R,5R,6R)-5-(2-(carboxymethoxy)acetamido)-3,4,6-trihydroxytetrahydro-2H-pyran-2-carboxylic acid, 2-(2-oxo-2-(((2R,3R,4R,5S,6R)-2,4,5-trihydroxy-6-(hydroxymethyl)tetrahydro-2H-pyran-3-yl)amino)ethoxy)acetic acid, and derivatives and modified derivatives thereof. In a preferred embodiment, the monosaccharide has a molecular weight of at least about 200 Daltons to about 500 Daltons.

Examples of disaccharides include, but are not limited to, N-acetylactosamine, N-acylactosamine, N,N'-diacetyl chitobiose, N,N'-diacyl chitobiose, hyaluronic acid disaccharide, keratin disaccharide, keratin sulfate disaccharide, chondroitin disaccharide, chondroitin sulfate disaccharide, dermatan disaccharide, dermatan sulfate disaccharide, heparin disaccharide, and derivatives thereof.

The oligosaccharides of the current invention encompass those molecules composed of three or more monosaccharide units linked together at any free hydroxyl in the ordinary sense, either in a linear or branched manner, and are usually composed of 3 to 30 monosaccharide units, but in some instances may contain thousands of monosaccharide units. In particular, the oligosaccharides of the present invention may further include those oligosaccharides that are isolated or synthesized wherein any C3, C4 or C6 free hydroxyl of an N-acyl-2-amino monosaccharide is substituted with a single monosaccharide, and any other C2, C3, C4 or C6 free hydroxyl may be further substituted with one or more monosaccharides. Examples of the oligosaccharide include those discovered in a wide range of organisms such as animals, plants (including seaweeds), insects, microorganisms, and others, and include, but are not limited to, homooligomers of N-acetylglucosamine, chitin, partially deacetylated chitin, and other N-acyl-2-amino monosaccharides, heterooligomers composed of two or more different monosaccharides independently selected from the groups consisting of glucose, galactose, mannose, glucosamine, fructose, N-acyl-2-amino monosaccharides, iduronic acid, neuraminic acid, and sialic acid so long as the reducing end unit is unprotected (i.e. contains a hemiacetalic hydroxyl) and contains an N-acyl-2-amino moiety. Other representative oligosaccharides include hyaluronic acid, keratin, keratin sulfate, chondroitin, chondroitin sulfate, dermatan, dermatan sulfate, heparin, N-acetyllactosamine oligosaccharide, N-acetylchitotriose, **N-**acetylchitotetraose, and N-acetylchitopentose, chitin, including partially deacetylated chitin, native N-glycan cores, high-mannose N-glycans, hybrid N-glycans, complex N-glycans, and derivatives thereof.

Examples of native N-glycan cores include, but are not limited to, Man3 and Man3F. Examples of high-mannose N-glycans include, but are not limited to, Man-5, Man-6, Man-7, Man-8, and Man-9. Examples of hydrid N-glycans include, but are not limited, those carbohydrates wherein the GlcNAc terminates one branch of the N-glycan core, and a mannose terminates another branch of the N-glycan core. Examples of complex N-glycans include, but are not limited to, GO-N, GO, GOF-N, GOF, GOFB, G1, G1F, G2, G2F, G2FB, G1S1(α2,3), G1S1(α2,6), G1FS1(α2,3), G1FS1(α2,6), G2S1(α2,3), G2S1(α2,6), G2FS1(α2,3), G2FS1(α 2,6), G2S2(α 2,3), G2S2(α2,6), G2FS2(α2,3), G2FS2(α2,6), G2F with 2-α-Gal, A3, G3, G3S3(α2,6), A4, G4, Lewis A, Lewis B, Lewis X, Lewis Y, sialyl Lewis A, and sialyl Lewis X. The nomenclature used to describe the N-glycans would be known to one skilled in the art. With the exception of the Lewis antigens, all native N-glycan cores, high-mannose N-glycans, hybrid N-glycans, and complex N-glycans contain a Man₃GlcNAc₂ core, which may be optionally fucosylated or xylosylated.

The term "modified carbohydrate" (or "modified derivative thereof") used herein may refer to those modified through any process of isolation, separation and purification from naturally-occurring sources and origins, those that have been enzymatically modified, those that have been chemically modified, those that have been modified by biochemical means, including microorganisms, wherein such modifications may comprise those known in the field of glycoscience, for example, hydrolysis, oxidation, reduction, esterification, acylation, amination, etherification, nitration, dehydration, glycosylation, phosphorylation, and sulfation.

Linear oligosaccharides such as chitin, including partially deacetylated chitin, hyaluronic acid, keratin, keratin sulfate, chondroitin, chondroitin sulfate, dermatan, dermatan sulfate, and heparin can achieve molecular weights as much as 5,000,000 Daltons. Preferred sizes of these oligosaccharides include, but are not limited to, those with molecular weights of at least about 500 Daltons, at least about 1,000 Daltons, at least about 1,500 Daltons, at least about 3,500 Daltons, at least about 5,000 Daltons, at least about 10,000 Daltons, at least about 20,000 Daltons, at least about 25,000 Daltons, at least about 33,000 Daltons, at least about 50,000 Daltons, and at least about 100,000 Daltons. In another embodiment, preferred sizes of these oligosaccharides include, but are not limited to, those molecular weights that are less than about 6,000 Daltons, and less than about 50,000 Daltons.

Branched oligosaccharides such as high-mannose N-glycans, hybrid N-glycans, and complex N-glycans have molecular weights that are typically under 10,000 Daltons. Preferred sizes of these oligosaccharides include, but are not limited to, those with molecular weights of from about 500 Daltons to about 10,000 Daltons. A more preferred size of these oligosaccharides is from about 800 Daltons to about 5,000 Daltons. A most preferred size of these oligosaccharides is from about 800 Daltons to about 4500 Daltons.

A preferred SM is GalNac, preferably TriGalNac or related molecules.

The sample (or a test sample) may be a sample suspected of containing an analyte. A sample suspected of containing an analyte may refer to a sample for which the content of the analyte is unknown or unconfirmed. For example, a sample from a human suspected of having a disease (and therefore suspected of having the disease-associated analyte in his/her sample), but not known to have the disease, may constitute a sample suspected of containing an analyte. Embodiments of the method may also be used for confirmatory or further testing of a sample (e.g. to confirm the presence or absence of an analyte in the sample, or to further determine an amount/relative amount of the analyte in the sample) that has already been evaluated earlier by another method. Embodiments of the method also provide for a control sample that may be used to obtain an indication on whether the method is correctly performed. A "control sample" is distinguished from the "sample" or "test sample" in that the contents of the analyte (presence, absence etc.) in the "control sample" is known. A "control sample" includes both negative and positive control samples.

An alternative sample is a sample provided from a product of a glycosylation reaction, for example in-vitro.

The sample may be obtained from any source. For example, the sample may be a biological sample, a pharmaceutical sample, an environmental sample, a food sample etc. In some embodiments, the sample comprises a biological sample. Examples of biological samples include, but are not limited to blood, serum, plasma, sputum, saliva, lavage fluid (e.g. bronchial lavage fluid, alveolar lavage fluid and bronchoalveolar lavage fluid), nasal fluid/swab/wash/aspirate, anterior nares fluid/swab/wash/aspirate, nasal mid-turbinate fluid/swab/wash/aspirate, pharyngeal fluid/swab/wash/aspirate, nasopharyngeal fluid/swab/wash/aspirate, oropharyngeal fluid/swab/wash/aspirate, tissue biopsy e.g. lung biopsy, cerebrospinal fluid, urine, faeces, stool, anal swab, semen, sweat, tears, processed fractions thereof and the like. Saliva samples may be obtained, for example, from the mouth, the throat include the deep throat, the posterior oropharyngeal, the oropharynx or the naso-oropharyngeal etc.

In some embodiments, the method comprises obtaining a sample. In some embodiments, the method comprises processing a sample prior to its provision to the substrate. Methods of processing different types of samples known in the art may be employed prior to their provision to the substrate. For example, methods of processing a biological sample may involve centrifugation to separate a sample into different fractions, cell lysis, extraction of DNA and/or RNA, disintegration and/or dissolving of sample, purification and/or sample concentration. In one embodiment, the method further comprises substantially dissolving/solubilizing the sample in a solvent to obtain a liquid/fluid sample.

In various embodiments, the method further comprises, after the sample applying step but prior to the detecting determining/detecting step, washing the substrate with a washing reagent to remove free molecules from the substrate. Advantageously, this can improve the accuracy of the detection of signal effected by the reporter agent to determine the presence or absence of said glycosylated analyte captured on said substrate by said capture protein. As unbounded and/or weakly bounded molecules (e.g., not containing the target analyte of interest) are removed, the likelihood of obtaining false positives (e.g., when the reporter agent is one that binds to an analyte) is also reduced.

The method may also further comprise, blocking the substrate with a blocking agent/buffer. The blocking step may be carried out prior to the sample applying step. For example, when the capture protein has been incubated with the sample prior to the sample applying step, the blocking step may be carried out prior to the sample applying step. For example, when the capture protein is immobilised on the one more or more test zones of the substrate prior to the sample applying step, the blocking step may be carried out after applying the capture protein to the test zones and before applying the sample. Advantageously, the blocking step may improve the specificity of an assay by reducing background interference and improving the signal-to-noise ratio. Examples of blocking agents/buffers include, but are not limited to, bovine serum albumin (BSA) e.g., at from about 1 to about 5% concentration, non-fat dry milk (NFDM) e.g., at from about 0.1 to about 3% concentration, fish gelatin, whole sera, polyethylene glycol (PEG), polyvinyl alcohol (PVA), and polyvinylpyrrolidone (PVP). The reporter agent may be any detectable agent that is capable of indicating/reflecting a presence/absence and/or an amount of analyte that is captured by the capture protein. For example, the reporter agent may bind to an analyte and the detection of a signal effected by the reporter agent on a substrate may indicate the presence of analyte that is captured by the capture protein on the substrate. For example, the reporter agent may also compete with an analyte for binding to a capture protein and detection of signal effected by the reporter agent on a substrate may indicate that little or no analyte is captured by the capture protein on the substrate. In various embodiments therefore, the reporter agent comprises an analyte binder or a competing binder.

In one embodiment, the reporter agent comprises an analyte binder or a binding protein that is capable of binding to the analyte. In various embodiments, the reporter agent/analyte binder is capable of recognising and/or binding to the analyte. In various embodiments, the reporter agent/analyte binder has binding affinity for the analyte. In various embodiments, the reporter agent/analyte binder is specific to the analyte. In various embodiments, the affinity between the reporter agent/analyte binder and the analyte is not more than about 10⁻⁶ M, not more than about 10⁻⁷ M, not more than about 10⁻⁸ M, not more than about 10⁹⁹ M, not more than about 10⁻¹⁰ M, not more than about 10⁻¹¹ M, not more than about 10⁻¹² M, not more than about 10⁻¹³ M, not more than about 10⁻¹⁴ M, not more than about 10⁻¹⁵ M or less than about 10⁻¹⁵ M. In some embodiments, the affinity between the reporter agent/analyte binder and the analyte is in the micromolar range, optionally in the low micromolar range. In some embodiments, the affinity between the reporter agent/analyte binder and the analyte is in the nanomolar range, optionally in the low nanomolar range. In some embodiments, the affinity between the reporter agent/analyte binder and the analyte is in the picomolar range, optionally in the low picomolar range. In some embodiments, the affinity between the reporter agent/analyte binder and the analyte is in the femtomolar range, optionally in the low femtomolar range. In one embodiment, the reporter agent comprises a competing binder or a binding protein that is capable of binding to the capture protein. In various embodiments, the reporter agent/competing binder is capable of recognising and/or binding to the capture protein. In various embodiments, the reporter agent/competing binder has binding affinity for the capture protein. In various embodiments, the reporter agent/competing binder is specific to the capture protein. In various embodiments, the affinity between the reporter agent/competing binder and the capture protein is not more than about 10⁻⁶ M, not more than about 10⁻⁷ M, not more than about 10⁻⁸ M, not more than about 10⁻⁹ M, not more than about 10⁻¹⁰ M, not more than about 10⁻¹¹ M, not more than about 10⁻¹² M, not more than about 10 ⁻¹³ M, not more than about 10⁻¹⁴ M, not more than about 10⁻¹⁵ M or less than about 10⁻¹⁵ M. In some embodiments, the affinity between the reporter agent/competing binder and the capture protein is in the micromolar range, optionally in the low micromolar range. In some embodiments, the affinity between the reporter agent/competing binder and the capture protein is in the nanomolar range, optionally in the low nanomolar range. In some embodiments, the affinity between the reporter agent/competing binder and the capture protein is in the picomolar range, optionally in the low picomolar range. In some embodiments, the affinity between the reporter agent/competing binder and the capture protein is in the femtomolar range, optionally in the low femtomolar range. The competing binder may be the same or different from the analyte.

In various embodiments, the reporter agent is detectable. In some embodiments, the reporter agent is detectable via a detectable analyte binder, competing binder or binding protein comprised therein. For example, an analyte binder, competing binder or binding protein may be directly detectable, or it may be indirectly detectable e.g. it is capable of e.g. interacting with a reactant to produce a detectable signal or forming a complex that generates a detectable signal. In some embodiments, the reporter agent is detectable via a label.

A label may be any molecule or moiety that facilitates detection of the reporter agent (and therefore detection of any analyte competing binder bound to the reporter agent). A label may be capable of effecting a detectable signal, including signals that are visible and/or signals that are detectable using suitable sensor or instrumentation. Suitable labels depend on the particular assay format and/or detection system and may include those that are well known to those skilled in the art. Many labels are commercially available and may be used in embodiments of the method. Common types of labels include optical labels (e.g. fluorescent, luminescent and/or light-scattering labels, colorimetric labels, coloured molecules such as colloidal nanoparticles, molecules that generate colours during a reaction), radioactive labels, magnetic and/or electrical labels, enzymes, ligands with specific bonds, microscopic vesicles detectable by acoustic resonance, and the like. Examples of a label include, but are not limited to, a dye, a fluorescent dye, a fluorophore, a luminophore, a chromophore, a chromatogen, a chemiluminescent compound, a catalyst, an enzyme (e.g. a peroxidase), an enzymatic reagent/substrate, a biotin, a radioisotope, a mass label, a charge label, a spin label, a receptor, a ligand, a nanoparticle (e.g., gold, silver, carbon nanotubes), a colloidal metallic particle, a colloidal non-metallic particle or other moiety known in the art that can be measured by an analytical method and combinations thereof. A label can be directly detectable by the eye and/or by suitable sensor/instrumentation, or it can be detectable as a result of subsequent processes. For example, a label in the form of a dye may be directly detectable by the eye and/or by suitable sensor/instrumentation, while a label in the form of an enzyme such as horseradish peroxidase (HRP) become detectable when it produces a colour change (that may correlate with the analyte level) upon reaction with a substrate such as 3,3', 5, 5" - tetramethylbenzidine (TMB).

In various embodiments therefore, detecting a signal effected by the reporter agent may be direct (e.g. by the eye and/or by suitable sensor/instrumentation) or indirect (e.g. through enzymatic reaction of a substrate by the reporter agent).

In one embodiment, the label comprises a fluorescent dye. In one embodiment, the fluorescent dye comprises an Alexa Fluor (produced by Invitrogen Corporation) based dye molecule. In one example, the fluorescent dye comprises Alexa Fluor 594. In one embodiment, the label comprises an enzyme that is capable of producing a colorimetric signal/readout. In one embodiment, the enzyme comprises a peroxidase. In one embodiment, the peroxidase comprises HRP. Advantageously, the use of HRP is convenient as it can produce a colorimetric signal/readout that is visible/discern ible by the naked/unaided eye. The optical density of the colorimetric signal/readout can further be quantified/measured spectrophotometrically. Furthermore, HRP is capable of amplifying a signal and increasing the detectability of a reporter agent. HRP is also stable, and relatively resistant to heat and organic solvent.

A label may be coupled/attached directly to an analyte binder, competing binder or binding protein in a reporter agent, or it may be coupled/attached indirectly to an analyte binder, competing binder or binding protein in a reporter agent through one or more bridges or secondary reporter agents. For example, a label may be coupled/conjugated directly to an analyte binder, competing binder or binding protein in a reporter agent. In one embodiment, a label in the form of a fluorescent dye is coupled/conjugated to a competing binder to form the reporter agent. For example, a label may be coupled to an analyte binder, competing binder or binding protein through a biotin-streptavidin bridge or biotin- avidin bridge, or through a secondary antibody. In one embodiment, an analyte binder or binding protein is tagged with biotin and allowed to interact with HRP- conjugated streptavidin (SA-HRP) to produce a reporter agent in the form of a HRP-labelled analyte binder, competing binder or binding protein coupled through a biotin-streptavidin bridge. Advantageously, an indirect coupling/attachment of a label to an analyte binder or binding protein through e.g. the use of one or more bridges or secondary reporter agents may enhance/amplify a signal and thereby improve the detection sensitivity and/or the limit of detection of the method. For example, biotin and streptavidin has a dissociation constant (Kd) in the femtomolar range and the strong affinity between the two molecules allow more analytes to be coupled successfully to a label for detection. In one embodiment therefore, the reporter agent comprises a label coupled to an analyte binder, competing binder or binding protein through a biotin-streptavidin or biotin-avidin bridge. Advantageously, the biotin-streptavidin and biotin-avidin systems not only enhance/amplify a signal, but the systems are also versatile and compatible with a wide variety of analyte binders or binding proteins and labels.

In various embodiments, the reporter agent comprises an analyte binder, competing binder or binding protein and a label coupled thereto. In various embodiments, the reporter agent may further comprise one or more bridges. In various embodiments, the reporter agent comprises an analyte binder, competing binder or binding protein coupled to a label through one or more bridges. In various embodiments, the analyte binder, competing binder or binding protein is tagged with a biotin or biotinylated. The analyte binder, competing binder or binding protein may be further coupled or fused to one or more partners or moieties, e.g. a maltose-binding protein (MBP) to increase biotin accessibility. In various embodiments, the label is coupled/conjugated to streptavidin or avidin. In various embodiments, the reporter agent comprises an analyte binder, competing binder or binding protein coupled to a label through a biotin-streptavidin or biotin- avidin bridge. It may be appreciated that a label may already be present in the reporter agent e.g. at the incubating/contacting step or the reporter agent may be devoid of a label and is coupled to a label e.g. during or after the incubating/contacting step. In some embodiments therefore, the method comprises providing a reporter agent comprising a label. In some embodiments, the method comprises a step of adding/coupling a label (e.g. a streptavidin- conjugated label or a streptavidin-conjugated label) to an analyte binder, competing binder or binding protein (e.g. a biotinylated analyte binder, competing binder or binding protein), and optionally incubating/contacting the two, to obtain a reporter agent comprising/coupled to a label or a labelled reporter agent. The label adding/coupling step may be performed before, during or after the sample incubating step. The label adding/coupling step may be performed prior to the step of incubating the reporter agent with the capture protein. In some embodiments, a label is incubated with the sample and the analyte binder, competing binder or binding protein to obtain a reporter agent comprising/coupled a label or a labelled reporter agent (e.g. through biotin-streptavidin or biotin-avidin mediated coupling of the label to the analyte binder, competing binder or binding protein).

In various embodiments, the method further comprises adding a reagent capable of producing a detectable signal upon interaction/reaction with the reporter agent after the applying step. Suitable reagents depend on the particular reporter agent (e.g. the particular analyte binder, competing binder or binding protein and/or the particular label). For example, where the reporter agent comprises a HRP label, the reagent may be a HRP substrate that gives a detectable (e.g. coloured) end product upon reaction with HRP. In some embodiments therefore, where the label comprises HRP, the method comprises adding a reagent selected from the group consisting of o-Phenylene Diamine (OPD), 2,2'-azino-bis(3-ethylbenzthiazoline-6-sulphonate (ABTS), 3-amino-9- ethylcarbazole (AEC), 4-Chloro-1 -Naphtol (4-CN), 3,3",5,5"-tetramethylbenzidine (TMB), 3,3'-DiAminoBenzimidine (DAB), 10-Acetyl-3,7-Dihydroxyphenoxazine (ADHP), Resazurin, Luminol, Uptilight tyramide and combinations thereof.

In various embodiments, the method comprises a step of adding a label (e.g. SA-HRP) prior or after the applying step and a subsequent step of adding a reagent (e.g. TMB) for expressing the signal.

In various embodiments, the signal/readout effected or produced by the reporter agent or label include an optical signal/readout (e.g. colorimetric signal/readout, fluorescence, iluminescence such as chemiluminescence, electrochemiluminescence and photoluminescence etc.), radioactivity, infrared radiation, resonance energy transfer (FRET), emission signal/readout, magnetic and/or electrical signal/readout, acoustic signal/readout and any other forms of signal/readout that can be produced by a label or an analyte binder, competing binder or binding protein as described herein. In some embodiments, the signal/readout comprises a colorimetric signal/readout. In some embodiments, the colorimetric signal/readout is visible/discernible/distinguishable to the naked/unaided eye. In some embodiments, the signal/readout comprises a fluorometric signal/readout. In some embodiments, the intensity of the fluorometric/colorimetric signal/readout correlates with the level/amount/concentration of the analyte in the sample. In some embodiments, the intensity of the fluorometric /colorimetric signal/readout positively correlates with the level/amount/concentration of the analyte in the sample i.e., the higher the intensity of the fluorometric /colorimetric signal/readout, the higher the level/amount/concentration of the analyte in the sample. For example, a darker or more intense colour, or a higher fluorescence intensity produced by a reporter agent comprising an analyte binder may indicate that a sample contains a higher level/amount/concentration of the analyte than a lighter/fainter or less intense colour or a lower fluorescence intensity. In some embodiments, the intensity of the of the fluorometric /colorimetric signal/readout negatively correlates with the level/amount/concentration of the analyte in the sample (e.g., in a competitive binding assay) i.e., the higher the intensity of the fluorometric /colorimetric signal/readout, the lower the level/amount/concentration of the analyte in the sample. For example, a darker or more intense colour, or a higher fluorescence intensity produced by a reporter agent comprising a competing binder (e.g., in a competitive binding assay) may indicate that a sample contains a lower level/amount/concentration of the analyte than a lighter/fainter or less intense colour or a lower fluorescence intensity.

In some embodiments, the signal/readout is quantifiable. In some embodiments, detecting a signal/readout effected or produced by the reporter agent or label comprises capturing the signal/readout with a camera and optionally analysing the captured image to e.g. measure/quantify the intensity of the signal/readout. The image analysis may be carried out, for example, by use of Imaged software or other suitable imaging software programs or algorithms.

In some embodiments, the capture protein of the invention is characterized in that does not comprise a transmembrane domain and/or intracellular domain of ASGPR1 or ASGPR2.

The capture protein is preferably immobilized to a solid support or substrate. Preferably such solid support or substrate is a plate, a microplate, a well-plate, a slide, a bead, a microbead or nanobead, CHIP or a sensor chip or sensor tip.

A capture protein according to the present invention comprises an amino acid sequence of SEQ ID NO: 1: 
QNSQLQEELRGLRETFSNFTASTEAQVKGLSTQGGNVGRKMKSLESQLEKQQKDLSEDHSSL
LLHVKQFVSDLRSLSCQMAALQGNGSERTCCPVNWVEHERSCYWFSRSGKAWADADNYCR
LEDAHLVVVTSWEEQKFVQHHIGPVNTWMGLHDQNGPWKWVDGTDYETGFKNWRPEQPDD
WYGHGLGGGEDCAHFTDDGRWNDDVCQRPYRWVCETELDKASQEPPLL),
 or SEQ ID NO: 2: 
AQLQAELRSLKEAFSNFSSSTLTEVQAISTHGGSVGDKITSLGAKLEKQQQDLKADHDALLFHL
KHFPVDLRFVACQMELLHSNGSQRTCCPVNWVEHQGSCYWFSHSGKAWAEAEKYCQLENA
HLVVINSWEEQKFIVQHTNPFNTWIGLTDSDGSWKWVDGTDYRHNYKNWAVTQPDNWHGHE
LGGSEDCVEVQPDGRWNDDFCLQVYRWVCEKRRNATGEVA
 or a sequence having at least 80%, 85%, 90%, 95%, 96%, 97%, 98%, or 99% sequence identity to SEQ ID NO: 1 or 2.

A capture protein according to the present invention may comprise an amino acid sequence of SEQ ID NO: 7: 
WSHPQFEKGGGSGGGSGGSAWSHPQFEKENLYFQGGSGSGSQNSQLQEELRGLRETFSNF
TASTEAQVKGLSTQGGNVGRKMKSLESQLEKQQKDLSEDHSSLLLHVKQFVSDLRSLSCQMA
 
ALQGNGSERTCCPVNWVEHERSCYWFSRSGKAWADADNYCRLEDAHLVVVTSWEEQKFVQ
HHIGPVNTWMGLHDQNGPWKWVDGTDYETGFKNWRPEQPDDWYGHGLGGGEDCAHFTDD
GRWNDDVCQRPYRWVCETELDKASQEPPLL),
 or SEQ ID NO: 8: 
WSHPQFEKGGGSGGGSGGSAWSHPQFEKENLYFQGGSGSGSAQLQAELRSLKEAFSNFSS
STLTEVQAISTHGGSVGDKITSLGAKLEKQQQDLKADHDALLFHLKHFPVDLRFVACQMELLHS
NGSQRTCCPVNWVEHQGSCYWFSHSGKAWAEAEKYCQLENAHLVVINSWEEQKFIVQHTNP
FNTWIGLTDSDGSWKWVDGTDYRHNYKNWAVTQPDNWHGHELGGSEDCVEVQPDGRWND
DFCLQVYRWVCEKRRNATGEVA
 or a sequence having at least 80%, 85%, 90%, 95%, 96%, 97%, 98%, or 99% sequence identity to SEQ ID NO: 7 or 8.

The invention is in part predicated upon the finding that the use of the above specific domain sequences as capture proteins in accordance with the invention is specifically sensitive and accurate for analyte detection, quantification and purification.

Preferably, in some embodiments of the invention the assay device is suitable for detecting a glycosylated analyte via direct or indirect ELISA. Alternatively, the assay device is suitable for detecting a glycosylated analyte via surface plasmon resonance.

In some embodiments, the CRD is located at an end or at an extremity of the capture protein. In some embodiments, the CRD is attached or coupled to an end or an extremity of an analyte binder or a binding protein in the capture protein. In some embodiments, the CRD is attached or coupled to a terminus of the analyte binder or binding protein for the analyte. In some embodiments, the CRD is attached or coupled to a C-terminus of the analyte binder or binding protein for the analyte. In various embodiments, the positioning of the CRD within the capture protein advantageously allows the capture protein to be immobilised onto the substrate in an optimal orientation, e.g. with the binding faces facing away from the substrate surface and/or towards the applied sample, such that the binding faces are available for contacting and binding to the analyte. Consequently, more capture proteins can bind the analytes successfully, and detectability of the analytes may be increased. The sensitivity of the assay/method may be increased and false negative results can be reduced. The limit of detection may also be improved. In some embodiments therefore, a plurality of capture agents is immobilised on the cellulose substrate in a substantially uniform orientation.

The method may further comprise applying/contacting a control sample to the substrate on which the capture protein is immobilized to obtain an indication on whether the method is correctly performed. The control sample may comprise a positive control sample, a negative control sample, or both. As may be appreciated, a detection or an immobilization of a positive control sample may indicate that the method is correctly performed, for example, a binding of the reporter and capture proteins to the analyte is successful e.g. during the incubation step and that the result obtained is not a false negative due to unsuccessful binding e.g. during the incubation step. No detectable immobilization of a negative control sample may indicate that the method is correctly performed, for example, there is no non- specific interaction and that the result obtained is not a false positive. In some embodiments, the method comprises processing the control sample prior to applying/contacting it with the substrate. As may be appreciated, methods of processing the sample, as herein disclosed before, may apply to the control sample.

In various embodiments, the method may be implemented as a system. In various embodiments, there is provided a system for detecting an analyte in a sample, the system comprising: an incubation mixture comprising said sample and a reporter agent configured to bind to said analyte; and/or a substrate configured to capture the analyte that is bound to said reporter agent, via a capture protein comprising a carbohydrate recognition domain (CRD), wherein said capture agent is: (i) present in the incubation mixture; and/or (ii) pre-immobilised on said substrate, and wherein the presence or absence of said analyte captured on said sustrate is determinable by detection via the reporter agent. In various embodiments, there is provided a system for detecting an analyte in a sample, the system comprising: a substrate configured to capture the analyte and reporter agent, via a capture protein comprising a CRD, wherein said capture agent is: (i) present in an incubation mixture comprising said sample and a reporter agent configured to bind to the capture protein; and/or (ii) pre-immobilised on said substrate, and wherein the presence or absence of said analyte captured on said substrate is determinable by detection via the reporter agent. In various embodiments, the system further comprises the incubation mixture comprising said sample and the reporter agent configured to bind to the capture protein comprising a CRD. In various embodiments, the system may further comprise one or more of the following: an image capturing device/unit/module for capturing an image of the substrate; a processing device/unit/module for processing data obtained from the image and a storage medium for storing instructions to analyse the data from the image. In one embodiment, all of these modules/units can be part of a single mobile/portable device e.g., a mobile phone, and the instructions are part of an application installed in the mobile/portable device.

In some embodiments, detecting and quantifying the bound target analyte comprises contacting the bound analyte with a reporter agent, such as a detection antibody that binds to a different epitope of the bound analyte. Various embodiments with regard to the use of reporter agents are provided herein elsewhere. In a preferred embodiment, a detection antibody or protein comprises a detectable label, or wherein the method comprises contacting the detection antibody with a secondary detection antibody comprising a detectable label. Such detectable signal is a colorimetric, chemiluminescent, or fluorescent signal.

Usually, a signal is measured using a plate reader to determine the concentration of the target protein based on a standard curve.

In some embodiments of the invention the solid support/substrate comprises a sensor chip surface configured for surface plasmon resonance detection or comprises a biolayer interferometry (BLI) sensor.

Surface plasmon resonance (SPR) is a label-free, real-time analytical technique used to detect and quantify molecular interactions by monitoring changes in the refractive index at a sensor surface. In an SPR-based method, a polarized light beam is directed at a thin metal film, typically comprising gold, deposited on a glass substrate. Under specific angular or wavelength conditions, the incident light excites surface plasmons-coherent electron oscillations-at the metal-dielectric interface. The resonance condition is sensitive to changes in the local refractive index, which occur when analyte molecules bind to ligands immobilized on the sensor surface.

In a typical embodiment, the analyte which is capable of being bound to a substrate via specific binding to the capture protein which is immobilized on the substrate (the sensor surface) through covalent or non-covalent means, including but not limited to amine coupling, thiol coupling, affinity capture (e.g., biotin-streptavidin interaction), or hydrophobic adsorption. Analyte molecules in solution are then flowed across the sensor surface under controlled flow and temperature conditions. Binding of analyte to the immobilized capture protein results in a measurable shift in the SPR signal, typically expressed in resonance units (RUs), which correlates with the mass of the bound analyte.

In preferred embodiments, the method further comprises regenerating the sensor surface by introducing a regeneration buffer, such as a low-pH or high-salt solution, capable of dissociating the bound analyte without removing the immobilized ligand. This enables multiple cycles of binding and analysis on a single sensor surface.

The SPR response may be recorded as a sensorgram, representing response units as a function of time. The shape and magnitude of the sensorgram can be used to derive kinetic parameters, including the association rate constant (ka), dissociation rate constant (kd), and the equilibrium dissociation constant (KD). These kinetic values are particularly useful for characterizing binding affinities in biological research, drug discovery, and diagnostic development.

In certain embodiments, the SPR system may comprise a microfluidic flow cell, an optical detection module (e.g., prism-based or waveguide-based coupling), and software capable of performing reference subtraction, baseline correction, and curve fitting. Sensor surfaces may be configured for single or multiplexed analysis, and may include reference channels to account for non-specific binding or bulk refractive index changes.

In additional embodiments, the SPR method may be used in combination with other analytical modalities, such as mass spectrometry, fluorescence detection, or electrochemical sensing, to enhance sensitivity, specificity, or multiplexing capability. The sensor chip may further comprise functional coatings or self-assembled monolayers to enhance binding specificity, reduce fouling, or allow for selective attachment of ligands.

In other embodiments, the invention relates to bio-layer interferometry (BLI) which is an optical, label-free analytical technique employed to monitor biomolecular interactions in real time. BLI measures the interference pattern of white light reflected from two surfaces: (i) an internal reference layer and (ii) an external layer comprising a biological recognition element immobilized on the surface of a fiber optic or sensor tip. Changes in the optical thickness of the biological layer due to binding events are detected as wavelength shifts in the reflected light spectrum.

In a typical embodiment, the sensor comprises a disposable optical fiber or biosensor tip, the distal end of which is functionalized with the capture protein. The sensor tip is immersed in a liquid sample containing the analyte of interest. Upon binding of the analyte to the immobilized capture protein, the optical thickness of the bio-layer increases, resulting in a measurable shift in the wavelength of reflected light. This wavelength shift is directly proportional to the mass of the bound analyte.

BLI operates without the need for microfluidics or continuous flow systems. Instead, individual sensors are sequentially or simultaneously dipped into wells of a microplate containing different solutions, such as running buffer, analyte, and regeneration buffer. This format allows for high-throughput operation, simplified instrumentation, and reduced risk of cross-contamination. In preferred embodiments, BLI instruments are configured for parallel detection across multiple sensor tips, thereby enabling multiplexed or comparative analyses across many binding pairs.

The output of a BLI experiment is typically presented as a binding curve (sensorgram) plotting wavelength shift (in nanometers) versus time. From this sensorgram, kinetic parameters including the association rate constant (ka), dissociation rate constant (kd), and equilibrium dissociation constant (KD) may be extracted using suitable curve-fitting algorithms. BLI systems are further capable of epitope binning, competition assays, and quantitation of active binding sites on ligand-coated surfaces.

In certain embodiments, the sensor surface may be functionalized with biotin, Ni-NTA, Protein A/G, streptavidin, or other affinity tags to facilitate oriented and stable immobilization of the ligand. Sensors may also include anti-fouling layers or surface passivation agents to reduce non-specific binding. Regeneration of the sensor surface may be achieved through dipping into low-pH, high-salt, or detergent-based solutions that dissociate the analyte without disrupting the ligand layer.

BLI provides several advantages, including its label-free nature, high reproducibility, and tolerance to complex sample matrices such as serum or lysate. The technology is particularly well suited to applications in antibody screening, protein-protein interaction studies, small molecule binding analysis, and quality control of biopharmaceutical products.

In additional embodiments, BLI may be integrated with robotic platforms or liquid-handling systems for automated, high-throughput kinetic screening. The optical detection module may also be configured to operate in environments with ambient light, due to the wavelength-based detection scheme, which enhances operational flexibility.

In some embodiments, the capture protein is immobilized on the sensor surface via covalent coupling, affinity capture, or biotin-streptavidin interaction.

In yet further preferred embodiments, the method comprises injecting the analyte sample over the sensor surface under controlled flow conditions; or inserting a ligand-modified sensor into an analyte solution while shaking (BLI).

According, in preferred embodiments, binding of the glycosylated target analyte to the immobilized capture protein is detected in real time by measuring changes in the refractive index at the sensor surface or by measuring changes in wavelength interference.

In some preferred embodiments, the changes in refractive index are reported as resonance units (RUs).

In some preferred embodiments, the method is further comprising regenerating the sensor surface by dissociating the bound analyte using a regeneration buffer.

It may be preferred that the quantification of the target analyte is based on comparison to a calibration curve derived from standard protein concentrations.

Kinetic parameters, including association and dissociation rates (ka and kd), and binding affinity (KD), are preferably determined from the real-time binding data.

Preferred is that the sensor surface is part of an SPR-based biosensor selected from the group consisting of Biacore^{™}, OpenSPR^{™}, and ProteOn^{™} systems.

In **a third aspect,** the invention pertains to an assay kit, comprising the assay device of the invention, and optionally:
(a) One or more buffers and/or reagents
(b) A first detection antibody and optionally a second detection antibody.

In **a fourth aspect,** the invention pertains a use of a capture protein recited in the previous aspects for quantifying the in-vitro glycosylation of a target molecule and/or for purifying a glycosylated target molecule.

In **a fifth aspect,** the invention pertains to a purification device for glycan modified analytes, comprising a capture protein which is composed of a soluble extracellular domain of Asialoglycoprotein receptor (ASGPR) 1 or 2, or a protein having at least 80% sequence identity to an extracellular domain of ASGPR1 or ASGPR2; and wherein
the capture protein comprises at least a Carbohydrate recognition domain (CRD) of ASGPR1 or ASGPR2;
wherein the capture protein is as defined in any one of the preceding claims.

The subject matter described is a purification device specifically designed for glycan modified analytes. This device includes a capture protein, which is a key component responsible for selectively binding to the target analytes. The capture protein is defined as being composed of the soluble extracellular domain of either Asialoglycoprotein receptor 1 (ASGPR1) or Asialoglycoprotein receptor 2 (ASGPR2). Alternatively, the capture protein may be a protein that has at least 80 percent, more preferably 85 percent, more preferably 90 percent, preferably 95 percent, more preferably 98 or 99 percent sequence identity to the extracellular domain of ASGPR1 or ASGPR2. Sequence identity refers to the degree of similarity between the amino acid sequence of the protein used in the device and the reference ASGPR1 or ASGPR2 extracellular domains, ensuring that proteins with minor variations but similar functional properties are also covered.

The capture protein must include at least a Carbohydrate Recognition Domain (CRD) of ASGPR1 or ASGPR2. The CRD is a specific region within the ASGPR proteins that is responsible for recognizing and binding to carbohydrate structures, particularly those present on glycan modified analytes. By incorporating the CRD, the device ensures high specificity and affinity for glycan structures, which are often found on therapeutic proteins, antibodies, or other biologically relevant molecules.

The advantage of this subject matter lies in its ability to provide a highly selective and efficient method for purifying glycan modified analytes. The use of the ASGPR extracellular domain, and specifically the CRD, allows for the targeted capture of analytes based on their glycan modifications, which is particularly valuable in biopharmaceutical manufacturing and research. This approach can improve the purity and yield of glycan modified products, reduce the presence of undesired contaminants, and streamline downstream processing. The inclusion of proteins with high sequence identity to ASGPR1 or ASGPR2 further broadens the applicability of the device, allowing for the use of engineered or variant proteins that retain the desired binding characteristics. Overall, this subject matter offers a robust and versatile solution for the purification of glycan modified analytes, addressing challenges associated with specificity, efficiency, and scalability in analytical and preparative workflows.

In a further embodiment of the device according to the fifth aspect, the capture protein is immobilized on a solid support, preferably a matrix or bead suitable for affinity purification.

In **a sixth aspect,** the invention pertains to a method purifying a glycosylated molecule, the method comprising:
(a) Providing the purification device of the invention;
(b) Contacting the capture protein with an unpurified sample containing the glycosylated molecules, preferably under conditions allowing for a specific binding of the glycosylated protein to the capture protein;
(c) Optionally, removing unbound material (washing step), preferably under conditions retaining bound glycosylated protein to the capture protein;
(d) Eluting the glycosylated protein from the capture protein under conditions that allow for a release of the binding interaction between the capture protein and the glycosylated molecule,
(e) Obtaining from the elute in (d) a purified composition of the glycosylated protein.

The method of purification is preferred, wherein the binding between glycosylated molecules and the capture protein is a specific binding of the CRD to the carbohydrate of the glycosylated molecule.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1****:** shows a vector map.
**Figure 2****:** shows results of an ELISA using the invention
**Figure 3****:** shows results of an ELISA using the invention
**Figure 4****:** shows results of an ELISA using the invention
**Figure 5****:** shows results of a plasmon surface resonance using the invention

The sequences show:
**SEQ ID NOs. 1 to 2 show sequences of capture proteins**
   SEQ ID NO. 1: 
WSHPQFEKGGGSGGGSGGSAWSHPQFEKENLYFQGGSGSGSQNSQLQEELRGLRETFSNF
TASTEAQVKGLSTQGGNVGRKMKSLESQLEKQQKDLSEDHSSLLLHVKQFVSDLRSLSCQMA
ALQGNGSERTCCPVNWVEHERSCYWFSRSGKAWADADNYCRLEDAHLVVVTSWEEQKFVQ
HHIGPVNTWMGLHDQNGPWKWVDGTDYETGFKNWRPEQPDDWYGHGLGGGEDCAHFTDD
GRWNDDVCQRPYRWVCETELDKASQEPPLL
   SEQ ID NO. 2: 
WSHPQFEKGGGSGGGSGGSAWSHPQFEKENLYFQGGSGSGSAQLQAELRSLKEAFSNFSS
STLTEVQAISTHGGSVGDKITSLGAKLEKQQQDLKADHDALLFHLKHFPVDLRFVACQMELLHS
NGSQRTCCPVNWVEHQGSCYWFSHSGKAWAEAEKYCQLENAHLVVINSWEEQKFIVQHTNP
 
FNTWIGLTDSDGSWKWVDGTDYRHNYKNWAVTQPDNWHGHELGGSEDCVEVQPDGRWND
DFCLQVYRWVCEKRRNATGEVA
**SEQ ID NO. 3 shows TTL-ASGPR1**
   SEQ ID NO. 3: 
MYRMQLLSCIALSLALVTNSWSHPQFEKGGGSGGGSGGSAWSHPQFEKENLYFQGGSGSGS
QNSQLQEELRGLRETFSNFTASTEAQVKGLSTQGGNVGRKMKSLESQLEKQQKDLSEDHSSL
LLHVKQFVSDLRSLSCQMAALQGNGSERTCCPVNWVEHERSCYWFSRSGKAWADADNYCRL
EDAHLVVVTSWEEQKFVQHHIGPVNTWMGLHDQNGPWKWVDGTDYETGFKNWRPEQPDD
WYGHGLGGGEDCAHFTDDGRWNDDVCQRPYRWVCETELDKASQEPPLL
**SEQ ID NOs. 4 - 6 show sequence of ASFPR H2b extracellular domain and its nucleic acid construct**
   SEQ ID NO. 4: 
GGCGGCGCCCAGCTGCAGGCCGAGCTGAGGAGCCTGAAGGAGGCCTTCAGCAACTTCAGCAGCACCCTGAC
CGAGGTGCAGGCCATCAGCACCCACGGCGGCAGCGTGGGCGACAAGATCACCAGCCTGGGCGCCAAGCTGGAGA
AGCAGCAGCAGGACCTGAAGGCCGACCACGACGCCCTGCTGTTCCACCTGAAGCACTTCCCCGTGGACCTGAGGTT
CGTGGCCTGCCAGATGGAGCTGCTGCACAGCAACGGCAGCCAGAGGACCTGCTGCCCCGTGAACTGGGTGGAGCA
CCAGGGCAGCTGCTACTGGTTCAGCCACAGCGGCAAGGCCTGGGCCGAGGCCGAGAAGTACTGCCAGCTGGAGA
ACGCCCACCTGGTGGTGATCAACAGCTGGGAGGAGCAGAAGTTCATCGTGCAGCACACCAACCCCTTCAACACCTG
GATCGGCCTGACCGACAGCGACGGCAGCTGGAAGTGGGTGGACGGCACCGACTACAGGCACAACTACAAGAACTG
GGCCGTGACCCAGCCCGACAACTGGCACGGCCACGAGCTGGGCGGCAGCGAGGACTGCGTGGAGGTGCAGCCCG
ACGGCAGGTGGAACGACGACTTCTGCCTGCAGGTGTACAGGTGGGTGTGCGAGAAGAGGAGGAACGCCACCGGC
GAGGTGGCC
   SEQ ID NO. 5: 
GGGGGCGCCCAGCTGCAGGCTGAACTGAGGAGCCTGAAGGAGGCCTTCAGCAACTTCTC
TAGCTCCACCCTGACCGAGGTGCAGGCAATCAGCACCCACGGAGGAAGCGTGGGCGATAA
GATCACCAGCCTGGGCGCCAAGCTGGAGAAGCAGCAGCAGGACCTGAAGGCTGACCACG
ATGCCCTGCTGTTTCACCTGAAGCACTTCCCAGTGGACCTGAGGTTTGTGGCCTGTCAGAT
GGAACTGCTGCACTCCAATGGCTCCCAGAGGACCTGTTGCCCCGTGAATTGGGTGGAGCA
CCAGGGCAGCTGTTACTGGTTCTCTCACAGCGGGAAGGCCTGGGCCGAGGCTGAGAAGT
ACTGTCAGCTGGAGAATGCCCACCTGGTGGTGATCAACTCATGGGAGGAACAGAAATTTAT
CGTGCAGCACACTAACCCTTTCAACACTTGGATTGGTCTGACCGATAGCGATGGAAGCTGG
AAGTGGGTCGACGGGACAGACTACCGCCACAATTATAAAAATTGGGCAGTGACCCAGCCAG
ACAATTGGCACGGGCATGAGCTGGGGGGGAGCGAGGATTGCGTGGAGGTGCAGCCCGAT
GGCAGATGGAACGACGACTTTTGCCTGCAGGTGTACAGATGGGTGTGCGAGAAGCGCAGA
AACGCTACCGGCGAGGTGGCC
   SEQ ID NO. 6:

WSHPQFEKGGGSGGGSGGSAWSHPQFEKENLYFQGGSGSGSAQLQAELRSLKEAFSNFSS
STLTEVQAISTHGGSVGDKITSLGAKLEKQQQDLKADHDALLFHLKHFPVDLRFVACQMELLHS
NGSQRTCCPVNWVEHQGSCYWFSHSGKAWAEAEKYCQLENAHLVVINSWEEQKFIVQHTNP
FNTWIGLTDSDGSWKWVDGTDYRHNYKNWAVTQPDNWHGHELGGSEDCVEVQPDGRWND
DFCLQVYRWVCEKRRNATGEVA

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Design of the Capture Protein

The ASGPR1 receptor has is transmembrane domain from AA 41-61 and the binding region of triGalNAc is estimated in the C-terminal regions between 230 and 291. In order to have a purification and study ligand, a plasmid for mammalian expression containing the region 62-291 of (ASGPR1 P07306 · ASGR1_HUMAN) was created. The TTL-Version have a Twinstrep-tag (sequence from (https://www.iba-lifesciences.com/support/scientific-faqs/), a subsequent TEV protease cleavage site and a 3xGS linker until pos. 62 of ASGPR1. The protein has a Kozak-sequence and a subsequent IL-2 signal peptide, which is in charge for signaling protein secretion and is cleaved off during protein expression. The Plasmid was created at Genscript in a pcDNA3.4 vector.

The TTL-ASGPR1 has the following sequence (SEQ ID NO. 3):

MYRMQLLSCIALSLALVTNSWSHPQFEKGGGSGGGSGGSAWSHPQFEKENLYFQGG
SGSGSQNSQLQEELRGLRETFSNFTASTEAQVKGLSTQGGNVGRKMKSLESQLEKQQKDLSE
DHSSLLLHVKQFVSDLRSLSCQMAALQGNGSERTCCPVNWVEHERSCYWFSRSGKAWADAD
NYCRLEDAHLVVVTSWEEQKFVQHHIGPVNTWMGLHDQNGPWKWVDGTDYETGFKNWRPE
QPDDWYGHGLGGGEDCAHFTDDGRWNDDVCQRPYRWVCETELDKASQEPPLL

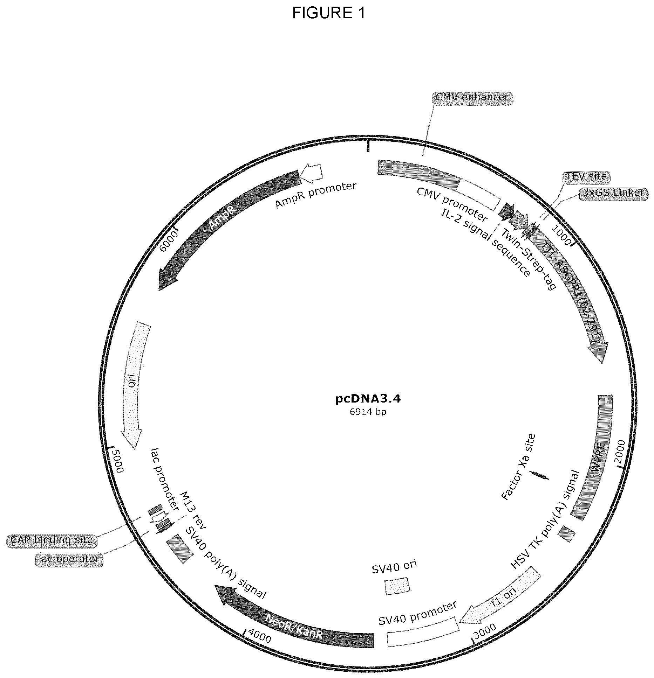

A plasmid map is provided in figure 1.

Furthermore, the following sequences for ASGPR H2b were obtained:

### Nomenclature:

| **Isoform** | **AA** | **Genbank** | **Uniprot** |
|---|---|---|---|
| H2a | 311 | NP_550434.1 | P07307-1 |
| H2b | 287 | NP_550435.1 | P07307-3 |
| H2c | 292 | NP_550436.1 | P07307-2 |

The sequence of ASGPR H2b extracellular domain was taken from uniport P07307 (database version of May 21, 2025) and reverse transcripted. The isoform P07307-3 is the isoform H2b, which is available intracellular. The extracellular domain was identified by kyte-doolittle plot and alphafold model. The residue 66 was chosen as a beginning point due to definetly end of the transmembrane helix marked by Q61 and H65. The construct was chosen from 117 to 287.

The sequence was codon optimized
Pasted Sequence (SEQ ID NO. 4): GC=65.64%, CAI=1.00 
GGCGGCGCCCAGCTGCAGGCCGAGCTGAGGAGCCTGAAGGAGGCCTTCAGCAACTTCAG
CAGCAGCACCCTGACCGAGGTGCAGGCCATCAGCACCCACGGCGGCAGCGTGGGCGAC
AAGATCACCAGCCTGGGCGCCAAGCTGGAGAAGCAGCAGCAGGACCTGAAGGCCGACCA
CGACGCCCTGCTGTTCCACCTGAAGCACTTCCCCGTGGACCTGAGGTTCGTGGCCTGCCA
GATGGAGCTGCTGCACAGCAACGGCAGCCAGAGGACCTGCTGCCCCGTGAACTGGGTGG
AGCACCAGGGCAGCTGCTACTGGTTCAGCCACAGCGGCAAGGCCTGGGCCGAGGCCGA
GAAGTACTGCCAGCTGGAGAACGCCCACCTGGTGGTGATCAACAGCTGGGAGGAGCAGA
AGTTCATCGTGCAGCACACCAACCCCTTCAACACCTGGATCGGCCTGACCGACAGCGACG
GCAGCTGGAAGTGGGTGGACGGCACCGACTACAGGCACAACTACAAGAACTGGGCCGTG
ACCCAGCCCGACAACTGGCACGGCCACGAGCTGGGCGGCAGCGAGGACTGCGTGGAGG
TGCAGCCCGACGGCAGGTGGAACGACGACTTCTGCCTGCAGGTGTACAGGTGGGTGTGC
GAGAAGAGGAGGAACGCCACCGGCGAGGTGGCC
Improved DNA[1] (SEQ ID NO. 5): GC=58.59%, CAI=0.94 
GGGGGCGCCCAGCTGCAGGCTGAACTGAGGAGCCTGAAGGAGGCCTTCAGCAACTTCTC
TAGCTCCACCCTGACCGAGGTGCAGGCAATCAGCACCCACGGAGGAAGCGTGGGCGATA
AGATCACCAGCCTGGGCGCCAAGCTGGAGAAGCAGCAGCAGGACCTGAAGGCTGACCAC
GATGCCCTGCTGTTTCACCTGAAGCACTTCCCAGTGGACCTGAGGTTTGTGGCCTGTCAG
ATGGAACTGCTGCACTCCAATGGCTCCCAGAGGACCTGTTGCCCCGTGAATTGGGTGGAG
CACCAGGGCAGCTGTTACTGGTTCTCTCACAGCGGGAAGGCCTGGGCCGAGGCTGAGAA
GTACTGTCAGCTGGAGAATGCCCACCTGGTGGTGATCAACTCATGGGAGGAACAGAAATT
TATCGTGCAGCACACTAACCCTTTCAACACTTGGATTGGTCTGACCGATAGCGATGGAAGC
TGGAAGTGGGTCGACGGACAGACTACCGCCACAATTATAAAATTGGGCAGTGACCCAG
CCAGACAATTGGCACGGGCATGAGCTGGGGGGGAGCGAGGATTGCGTGGAGGTGCAGC
CCGATGGCAGATGGAACGACGACTTTTGCCTGCAGGTGTACAGATGGGTGTGCGAGAAGC
GCAGAAACGCTACCGGCGAGGTGGCC
The following amino acid sequence (SEQ ID NO. 6) is used:

[129]    WSHPQFEKGGGSGGGSGGSAWSHPQFEKENLYFQGGSGSGSAQLQAELRSLKEAF
SNFSSSTLTEVQAISTHGGSVGDKITSLGAKLEKQQQDLKADHDALLFHLKHFPVDLRFVACQM
ELLHSNGSQRTCCPVNWVEHQGSCYWFSHSGKAWAEAEKYCQLENAHLVVINSWEEQKFIV
QHTNPFNTWIGLTDSDGSWKWVDGTDYRHNYKNWAVTQPDNWHGHELGGSEDCVEVQPDG
RWNDDFCLQVYRWVCEKRRNATGEVA*

### Example 2: ELISA Assays using the Capture Protein of the Invention.

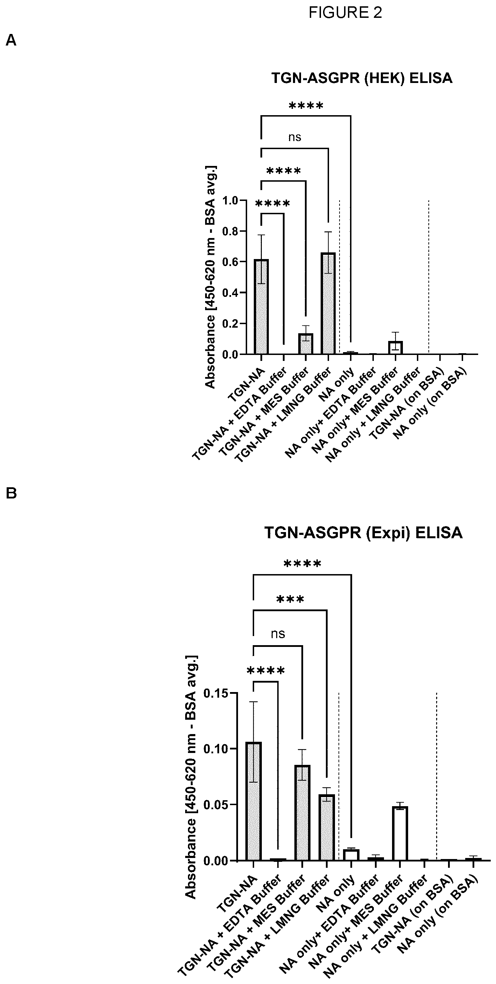

Results of the TriGaINAc-ASGPR ELISA are provided in Figure 2A and Figure 2B. Biotinylated TriGaINAc (TGN) was pre-incubated with Neutravidin-HRP (NA) and then added to coated TTL-ASGPR derived from either Expi293F (Expi - Figure 2A) or HEK293 (HEK) cells (Figure 2B). Absorbance was calculated by substracting the 450 from the 620 nm signal and additionally substracting the BSA coating average signal from the sample signals.

Binding was performed in different buffers. Dilution/TGN-NA and NA-only/Washing Buffer: 10mM HEPES, 150mM NaCl, 10mM CaCl2, pH7.4; EDTA-Buffer: 10mM HEPES, 150mM NaCl, 10mM EDTA, pH7.4; MES Buffer: 10mM MES, 150mM NaCl, 10mM CaCl2 pH 5.8; LMNG Buffer: 10mM HEPES, 150mM NaCl, 10mM CaCl2, 0.001% LMNG pH7.4.

Statistic analysis was done via one-way ANOVA with Šídák's multiple comparisons test, P > 0.05 (ns), P ≤ 0.05 (*), P ≤ 0.01 (**), P ≤ 0.001 (***) and P ≤ 0.0001 (****), n=3.

### Example 3: Further ELISA Assays using the Capture Protein of the Invention

Capture protein of the invention was coated in wells over night at 4°C with 100 ng/well in the respective coating buffer. Between every step, each well was washed three times with 300 µL/well washing buffer. To reduce unspecific binding, 300 µL/well blocking buffer was applied for 1h at RT. Subsequently, samples were added at different concentrations in dilution buffer. After incubation for 2h, detection antibodies (HRP) were applied for 1h at RT. Finally, enzymatic reaction was initiated by application of 100 µL/well tetramethylbenzidine (TMB) and hydrogen peroxide (H2O2) in a 1:1 ratio (v/v). Incubation time was chosen, until a clear signal was visible and stopped by addition of 100 µL/well 0.5 M sulfuric acid (H2SO4). Absorbance was measured at a wavelength of 450 nm and 620 nm using the VantaStar plate reader. The following table summarizes antibodies used for different constructs. A HEPES based buffer was used with Calcium. By the replacement of Ca2+ with EDTA, calcium-dependent binding was prohibited since ions are chelated. Thus, this setup was additionally used to ensure, that binding of sample to ASGPR was calcium-dependent.

| **Sample** | **Detection antibodies** |
|---|---|
| Siltuximab-TGN variants | anti-human HRP (1:1000) |
| Tocillizumab-TGN variants | |
| sIL6R-TGN variants | anti-His-HRP (1:5000) |
| Nb_IL6-TGN variants | anti-His-HRP (1:5000) |

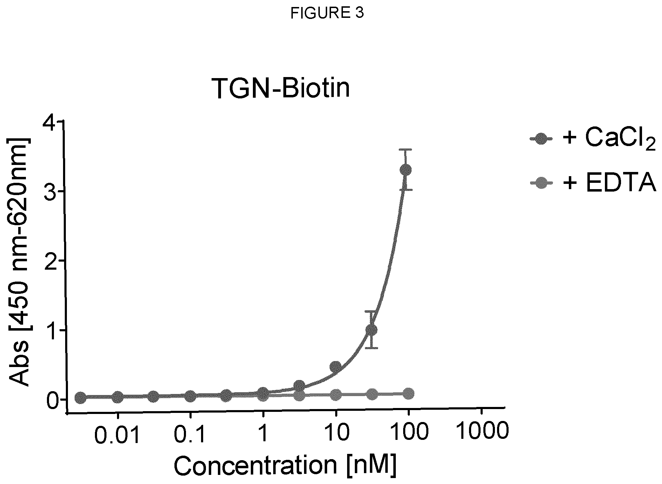

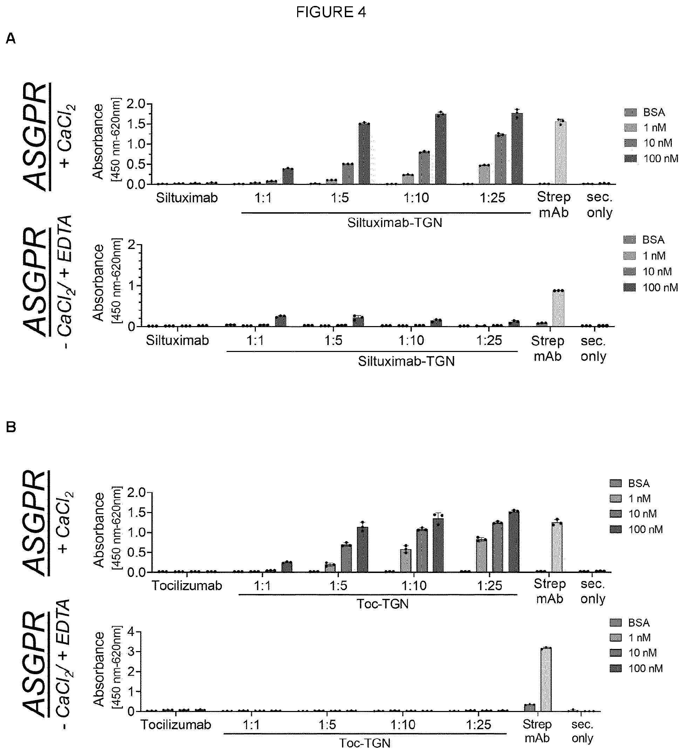

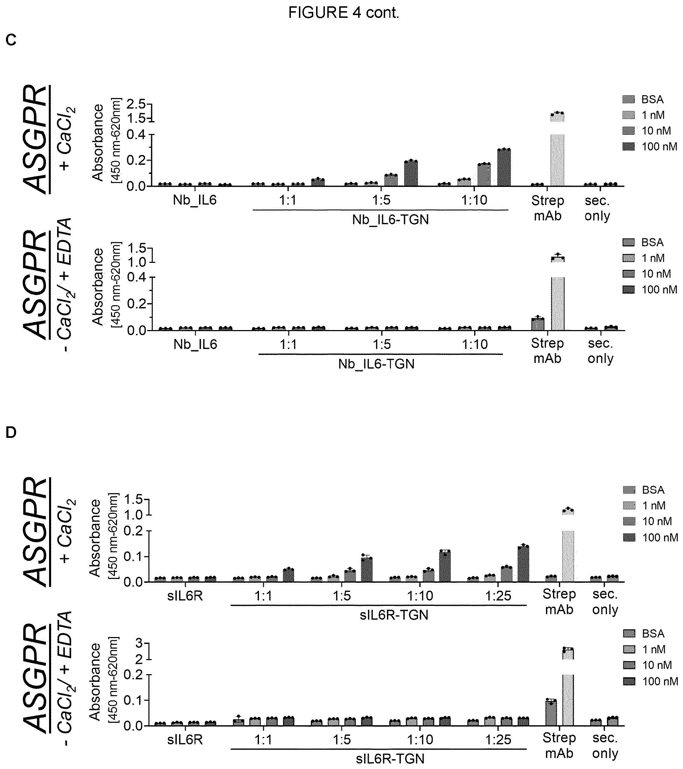

The results are shown in figures 3 and 4.

### Example 4: Surface Plasmon Resonance Detection of Capture Protein bound Analyte:

To quantify binding parameters of molecular interactions, SPR measurements were performed using the Biacore S200. Both IL6 as well as ASGPR were covalently immobilized using amine coupling on a CM5 chip. Briefly, the surfaces of all four
flow cells were activated for 10 min with a 1:1 mixture of 0.1 M N-hydrosuccinimide (NHS) and 0.1 M 3-(N,N-dimethylamino)-propyl-N-ethylcarbodiimid (EDC) at a flow rate of 10 µL/min. The ligand was applied at a concentration of 15 µg/mL. Flow cell 1 was left blank as a reference. All surfaces were finally blocked with a 7 min injection of 1 M Ethanolamine.

Binding of glycan-based BioDegs to ASGPR was analyzed by surface plasmon resonance (SPR) using a Biacore T200 system. ASGPR was immobilized on a CM5 sensor chip via standard amine coupling. BioDegs were injected as analytes at increasing concentrations for 300 s (association phase), followed by a 1200 s dissociation phase. All experiments were performed at 25 °C in HEPES-buffered saline with CaCl2.

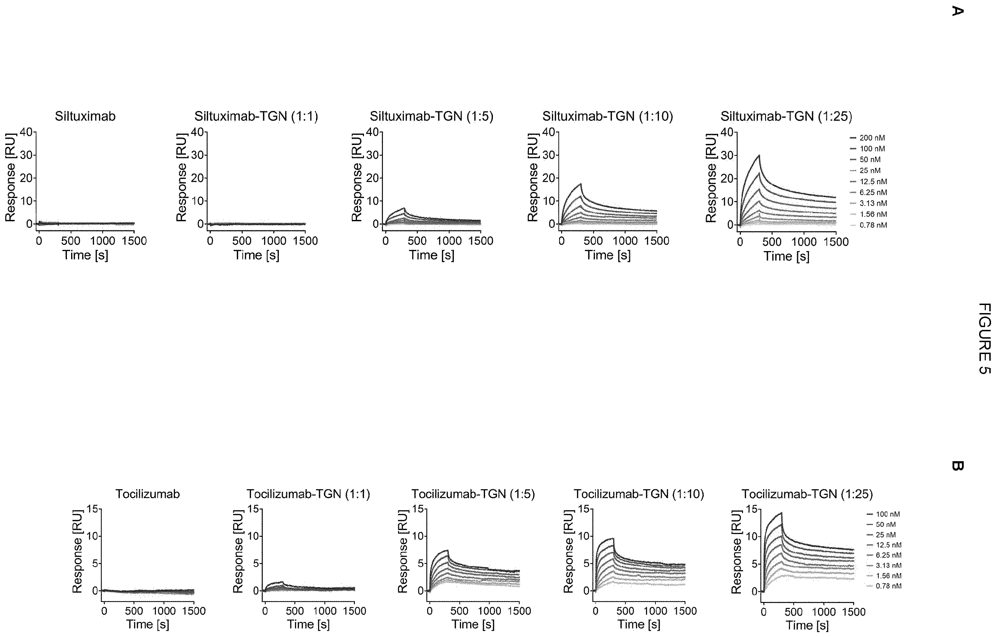

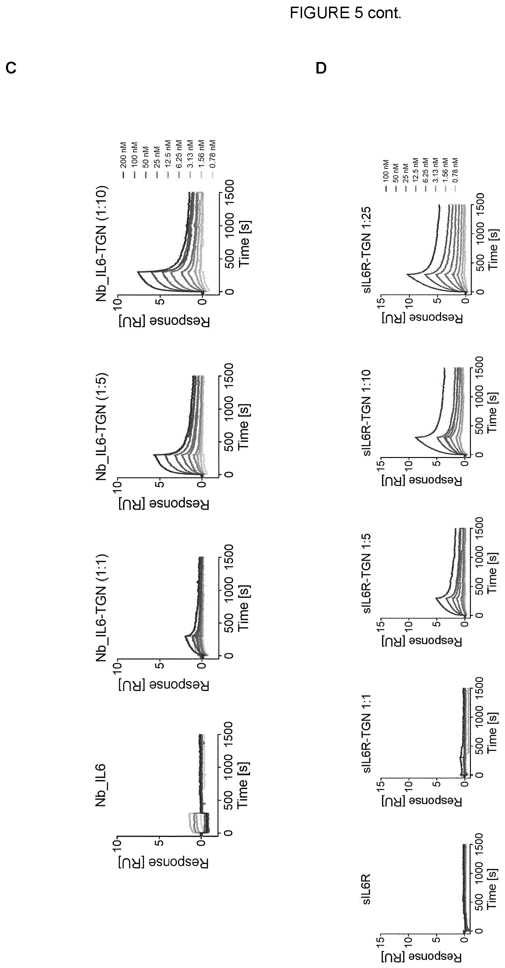

Results are provided in Figure 5.

## Claims

1. **An assay device for the detection and/or quantification of glycan modified analytes,** comprising a capture protein which is composed of a soluble extracellular domain of Asialoglycoprotein receptor (ASGPR) 1 or 2, or a protein having at least 80% sequence identity to an extracellular domain of ASGPR1 or ASGPR2; and wherein
the capture protein comprises at least a Carbohydrate recognition domain (CRD) of ASGPR1 or ASGPR2.

2. The assay device of claim 1, wherein the capture protein is immobilized to a solid support (or substrate), preferably wherein the solid support is a plate, a microplate, a well-plate, a slide, a bead, a microbead or nanobead, CHIP or a sensor chip or sensor tip.

3. The assay device of claim 1 or 2, wherein the capture protein comprises an amino acid sequence selected from the group consisting of SEQ ID NO: 1, 2, 7 or 8, or a sequence having at least 80% sequence identity to SEQ ID NO: 1, 2, 7 or 8.

4. The assay device of any one of claims 1 to 3, wherein the assay device is suitable for detecting a glycosylated analyte via direct or indirect ELISA; or wherein the assay device is suitable for detecting a glycosylated analyte via surface plasmon resonance or biolayer interferometry.

5. **A method for detecting and/or quantifying the glycosylation of a target analyte,** the method comprising:
(a) Providing the assay device of any one of claims 1 to 4,
(b) Contacting the capture protein with an analyte sample;
(c) Optionally, removing unbound material (washing step);
(d) Detecting the presence and the amount of analyte bound to the capture protein.

6. The method of claim 5, further comprising blocking non-specific binding sites on the solid support prior to contacting the sample, using a blocking buffer comprising bovine serum albumin (BSA), casein, or a detergent.

7. The method of claim 5 or 6, wherein detecting and quantifying the bound target analyte comprises contacting the bound analyte with a detection antibody that binds to a different epitope of the bound analyte.

8. The method of claim 7, wherein the detection antibody or protein comprises a detectable label, or wherein the method comprises contacting the detection antibody/protein with a secondary detection antibody comprising a detectable label.

9. The method of claim 5, wherein the solid support comprises a sensor chip surface configured for surface plasmon resonance detection or comprises a biolayer interferometry sensor.

10. The method of claim 5 or 9, further comprising injecting the analyte sample over the sensor surface under controlled flow conditions; or inserting a ligand-modified sensor into an analyte solution while shaking

11. The method of claim 5, 9 or 10, wherein the binding of the glycosylated target analyte to the immobilized capture protein is detected in real time by measuring changes in the refractive index at the sensor surface or by measuring changes in wavelength interference.

12. **An assay kit,** comprising the assay device of any one of claims 1 to 4, and optionally:
(a) One or more buffers and/or reagents
(b) A first detection antibody and optionally a second detection antibody.

13. **A use** of an assay device of any one of claims 1 to 4 or a method of any one of claims 5 to 11 for quantifying the in-vitro glycosylation of a target molecule.

14. **A purification device for glycan modified analytes,** comprising a capture protein which is composed of a soluble extracellular domain of Asialoglycoprotein receptor (ASGPR) 1 or 2, or a protein having at least 80% sequence identity to an extracellular domain of ASGPR1 or ASGPR2; and wherein
the capture protein comprises at least a Carbohydrate recognition domain (CRD) of ASGPR1 or ASGPR2;
wherein the capture protein is as defined in any one of the preceding claims.

15. **A method purifying a glycosylated molecule,** the method comprising:
(a) Providing the purification device of claim 14;
(b) Contacting the capture protein with an unpurified sample containing the glycosylated molecules, preferably under conditions allowing for a specific binding of the glycosylated protein to the capture protein;
(c) Optionally, removing unbound material (washing step), preferably under conditions retaining bound glycosylated protein to the capture protein;
(d) Eluting the glycosylated protein from the capture protein under conditions that allow for a release of the binding interaction between the capture protein and the glycosylated molecule,
(e) Obtaining from the elute in (d) a purified composition of the glycosylated protein.

16. The method of claim 15, wherein the binding between glycosylated molecules and the capture protein is a specific binding of the CRD to the carbohydrate of the glycosylated molecule.
